**Europäisches Patentamt**

**European Patent Office**

**Office européen des brevets**

(19)

(11) Publication number: **0 050 763**
**A2**

(12) # EUROPEAN PATENT APPLICATION

(21) Application number: **81108056.3**

(22) Date of filing: **08.10.81**

(51) Int. Cl.³: **A 61 F 1/22**

(30) Priority: **28.10.80 AR 283040**

(43) Date of publication of application:
**05.05.82 Bulletin 82/18**

(84) Designated Contracting States:
**AT BE CH DE FR GB IT LI LU NL SE**

(71) Applicant: **Bicer, Demetrio**
**Warnes 2682**
**Buenos Aires(AR)**

(72) Inventor: **Bicer, Demetrio**
**Warnes 2682**
**Buenos Aires(AR)**

(74) Representative: **Graf, Helmut**
**Greflinger Strasse 7 Postfach 382**
**D-8400 Regensburg(DE)**

(54) Cardiovascular valvular prosthesis and manufacturing process of same.

(57) The invention refers to a cardiovascular pros-
thesis, of the type that comprises a suture textile ring
arranged surrounding the perimetral groove of a support on
which an occluding disc is assembled.

The invention is characterised by the fact that said
support is constituted by an integral piece, of annular shape,
on one of the edges of which is projected a radially directed
support arm that ends in a terminal directed toward the
internal portion of same, said arm is complemented with a
pair of projections symmetrically arranged with respect to
same, that face the opposite edge of said annular piece
according to a virtual cord of same determined between the
diameter of the piece and the beginning of the above
mentioned radially directed arm; between the terminals of
said arms and of said projections the occluding disc is
adapted, free to rotate, that on the surface facing the above
mentioned arm has a circular depression the edge of which
forms a support step of the above mentioned terminal of said
arm; the textile ring adapted in the outside portion of said
perimetral groove of the support is constituted by a length of
a tubular piece of fabric that has its perimetral edges joined
by means of longitudinal seam disposed against said
groove.

Fig. 1

**0050763**

CARDIOVASCULAR VALVULAR PROSTHESIS AND MANUFACTURING PROCESS
OF SAME

The present invention refers to a cardiovascular valvular prosthesis and the process to manufacture same, said prosthesis with a totally novel conception, advantageously solves some of the problems encountered in the cardiac valves known at present.

Persistent problems of reliability have taken the regulating offices for the use of said prosthesis in some countries, specifically in United States of America, to adopt tiresome analysis requirements with the object to eliminate possible disadvantages to the user and the danger arising from eventual complications subsequent to their implantation. The final object will consist of obtaining a pertinent regulation for the approval prior to the commercialization of said prosthesis.

Although at present different types and models of cardiac valves exist in the world, some of them, once they have been implanted on the patient, in the course of time have originated anomalies and problems that in certain cases have caused the death of the patient. It was evidenced in one particular case, published in "BRIEFCASE", edited by Regulatory Associates Inc., in May 1980, and that was notified to the manufacturer of the prosthesis (Shiley Lab. of Irvine, California, U.S.A.) by Dr. Viking Björk, an outstanding cardio-surgeon of the Karolinska Institute of Stockholm, Sweden, of a patient that after 18 months of being implanted, the valve support had sustained a fracture, also pointing out a cause of a similar failure found in the clinical evaluation of a patient.

With reference to this matter, the Executive Secretary of the Classification Panel of Cardiovascular Design in the United States of America, has stated that the above mentioned incident is only one of many other situations and that the majority of the valves that have failed is due to durability problems. A defective design, the lack of attention in the engineering details, etc. have contributed to this situation.

The valve under consideration corresponded to a type protected in the United States of America by a patent of invention of October 1972, that has an occluding disc held between spaced elements that form, in a support ring, a kind of crossbars with folded portions, the longest of said crossbars having the middle portion approximately coincident with the virtual diameter of the occluding disc, both being of a substantially "U" shape and the angle of maximum opening of said disc being of approximately 62º.

Said disc is constituted by an acetal resin formed by polymerization of formaldehyde with a high cristallization structure.

In the Sorin valve, the opening angle of the occluding disc is in the order of 61 to 68º. The supports of said valve have a similar shape as that of the valve known as the Shiley valve.

Said durability problems of the valves are attributed to mechanical characteristics in the manufacture of the supports that are generally used as, due to the shape of same, they are cast, or in other cases, require welding for fixing to the "U" shaped transversal means that support the disc.

In any of the above mentioned cases, it is almost impossible to assure that said supports do not have weak areas, either because the casting does not offer the same guaranties as the machined material or because the welded areas may crack due to the quantity of movements to which they are submitted.

It must be taken into consideration that said valve must endure an average of 45 million cycles per year, said figure, multiplied by the minimum life expectation, notably increases said figure.

Contact between surfaces should be limited in every way possible, in order to avoid damage to the blood through compression that could lead to irreparable clinic consequences such as microembolism, forming of clots through adherence, etc.

Among other conditions is that of obtaining the greatest free circulation area possible and offer the least resistance to the passage of the blood flow. In the case of the above mentioned valves, the support means of the occluding disc, the opening angle of which does not reach to 70º, are interpolated in the flow passage which they divide offering hemodynamic resistance and the possibility of creating turbulence.

Another matter to be considered is the textile ring that fixes the valve. Same is usually formed by sewing the ends to give the adequate form, this implies having a transversal suture, with the risk of loosening said joining filaments due to failure of same or fatigue during its use.

Likewise, in the prosthesis generally used the occluding disc is not radiopaque, that does not allow its visualization for control by means of X-rays in the patient's clinic follow-up and through said means to effect a correct cardiac catheterism.

All the above mentioned disadvantages have been solved with the valvular prosthesis of the invention, the concept of which is thus completely novel and the results of which have been optimum. The evaluation of said prosthesis has been effected by the Biomedical Engineering and the Mechanical Engineering Departments of the Tulane University, in New Orleans, United States of America, said evaluation was carried out in an environment that simulates the geometrical characteristics as well as the dynamics of the human left ventricle, using a fluid similar to blood. The valve prototypes submitted for evaluation that correspond to the invention are in the mitral position and in the aortic position in a chamber especially designed for said type of study.

Therefore, it has been found that, as regards the quality of the material used for the support of the occluding disc as well as the particular shape of same and as a result of its manufacturing process the unit presents unusual characteristics in the physical, mechanical, chemical and other general performances that make same practically irreplaceable in the manufacture of this type of prosthesis.

Thus it has been found that said support is practically inert to solutions, acids, or alkalis at room temperature.

Said support is not obtained by casting or by welding the supports of the occluding disc (as mentioned above, said system is used at present by manufacturers of this type of valve, with the above mentioned structural problems). With the manufacturing process of the invention, the morphologic structure of the alloy used is maintained without altering the position of the crystals and thus keeps the physical as well as the chemical characteristics intact.

Furthermore, due to the special shape and position of the support means of the occluding disc, same not having radial lateral arms, or bulkiness at the ends or bendings, they offer a larger free passage area without obstacles in the central portion, thus obtaining a flow with a minimum resistance to the passage of fluid and practically eliminating the possibility of causing turbulence.

Another advantage in this assembly is that the opening of the occluding disc varies between 85º and 90º, with which

0050763

characteristics low profile and minimum hemodynamic resistance is obtained.

Also the shape of the occluding disc when in an open position fixes a laminar flow against the faces of same and said disc in a closed tangential contact allows a small peripheric insufficiency between same and the support that, without already verified clinic consequences, allows the washing of the internal surface of the support with the same blood through the action of the blood flow and the non-static accumulating of blood during the systole period with the closed valve.

Another advantage is the fact that the occluding disc is manufactured with carbon substrate (graphite) with a mixture in the order of 5% of tungsten that is perfectly visible in X-rays and allows the clinic follow-up control of the patient. Said material of the disc is biocompatible with blood and is thrombo-resistant.

Another advantage is established by the construction and fixing of the textile ring or fixing hoop of the prosthesis, which does not present transversal sutures or seams for joining its ends, being effected in one sole piece and thus offers higher resistance and prevents the risk of breakage by loosening of said threads or failure of same or fatigue during use, with the consequent risk of the loosening of the valve. It must be taken into account that the prosthesis, in its position, performs a slight movement on the commissural stitches as a consequence of the difference of the pressure between the systole and the diastole.

Another very important advantage of the invention is provided by the fact that the occluding disc, due to its special assembly, con freely rotate within its support. For this reason during its operation said effect allows an alternate contact of the surfaces of the disc with the support arms, avoiding that same should be exposed to wear in one only point, thus distributing the portions submitted to work and wear by friction or fatigue.

It is also pointed out that, with this particular conception, the fixing textile ring may be freely rotated over the housing groove in the support, that renders the possibility at the surgeon's decision, to carry out this operation to correct in situ any variation in the position of the valve that he may consider advisable during the implantation operation and once same is fixed by means of sutures. This eliminates the need to withdraw the sutures to reset the valve when the position of same must be corrected, which originates the consequent complications of loss of time and the eventual surgical risk for the patient.

Finally, it can be stated that the outcome of all these advantages is the obtainment of a valve that offers the most excellent clinical expectations, reduces risks and the possibility of embolism and its consequences, hemodynamic turbulences and their clinical complications to the extent that, even by using non thrombogenic materials acceptable in the manufacture of same, the use of anticoagulant drugs can be avoided in the post-operation treatment of the patient (that at present constitutes a constant preoccupation for the patient as said drugs are injectable and their application is periodical and extends during the patient's life time, with the consequent discomfort and trauma).

The other characteristics and advantages of the invention may be appreciated from the specification that, for better clearness and understanding, has drawings attached that represent the cardiovascular valvular prosthesis in its preferred embodiment, all of the foregoing as a non-restrictive example of its scope, in which:

Figure 1 is a perspective view of the cardiovascular valvular prosthesis of the invention, showing same in its maximum opening position, that allows to appreciate its characteristics as a whole.

Figure 2 is a perspective view of the disassembled valvular prosthesis, in which the basic component pieces may be observed.

Figure 3 is an elevated front view of the prosthesis, in the closed position, shown from the fluid inlet side through said prosthesis.

Figure 4 is a transversal section view of the prosthesis shown in the closed position of the previous figure in which the arrow points to the angular displacement of the occluding disc toward said closed position.

Figure 5 is a front view, similar to that of Figure 3, that shows the opening position of the occluding disc and, finally:

Figure 6 is a sectional view, similar to that of Figure 4, that shows the opening position of Figure 5, the arrow indicates the angular displacement of the occluding disc toward said opening position.

In the different figures the same reference numbers indicate equal or corresponding parts, having pointed out with letters the unit of several elements.

As may be observed from the drawings, the cardiovascular valvular prosthesis of the invention basically comprises three pieces properly connected to each other; as mentioned above, a support a which comprises a substantially rigid body manufacured with a special biocompatible alloy based on a high grade cobalt; an occluding disc b, assembled with angular displacement possibilities on said support a, manufactured from isotropic pyrolitic carbon with tungsten radiopaque incrustations, also biocompatible and thromboresistant, at the same time of great resistance to degrading by use and fatigue within the biologic means, the particular outline of which in the opening position, provides the maintenance of a laminar flow

with a minimum hemodynamic resistance; and a textile hoop or ring c manufactured in one only piece, that is irremovably adapted on support a and by means of sutures constitutes the fixing means of the assembly.

The above mentioned support a is constituted by an integral machined piece conforming an annular portion 1 in the external face of which a circunferencial groove 2 is defined adapted to the above mentioned textile ring c.

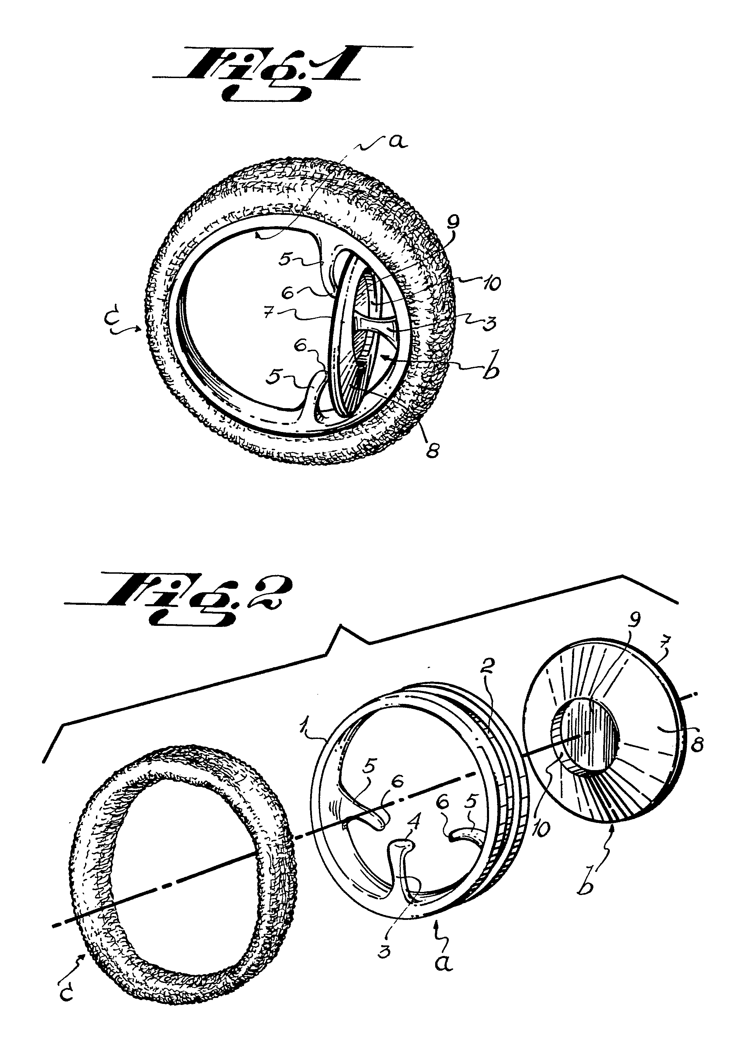

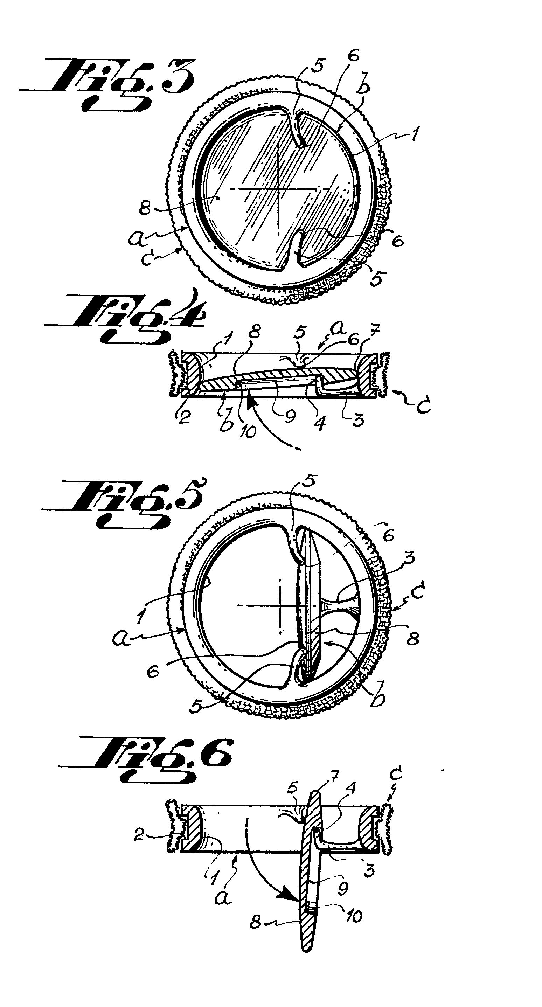

Toward the inner portion of said annular portion 1 is projected from an edge of same and in a radial direction a support arm 3 that ends in a terminal 4 which at a distance from the center of said annular portion 1 projects toward the inner portion, that is to say, toward the opposite face of that of the position of said arm 3, as may be observed in Figures 2, 4 and 6.

On both sides of said support arm 3, equidistant from same and projecting from the opposite edge of the annular portion 1 a set of support arms 5 are projected according to a virtual cord displaced with respect to the diameter of the annular portion 1, said arms 5 being shaped in such a way that their terminals 6 define the support means of the occluding disc b placed between same and the terminal 4 of the intermediate arm 3.

Thus, said support arms 3 and 5 being separated from each other and in different planes of the annular portion 1, as may be observed in Figures 4 and 6, the occluding disc b is adapted in a floating position same with three only tangential contact points with those corresponding to the terminals 4 and 6 of said support arms and with the possibility, aside from its angular displacemente in the manner shown by the arrows in Figures 4 and 6, of rotation as mentioned above.

As may be observed from the foregoing figures, due to the particular shape of the support arms 3 and 5 and the displaced position of the latter with reference to the diameter of the annular body 1 of support a, the occluding disc b leaves a substantially greater free area in its opening position that determines a flow (Figures 1 and 5) with a minimum hemodynamic resistance of said occluding disc b.

The profile of disc b shows a rounded perimetral edge 7, with divergent sides 3 that correspond to the convexity of the faces of same, the one placed facing the intermediate support arm 3 having a central depression 9 the internal edge of which, in the closed position, defines a step 10 against which terminal 4 of said arm 3 abutts, said terminal defines a pin, as may be observed in Figures 4 and 6.

The external perimetral groove 2 of support a adapts the textile ring c that is constituted by one single piece of "Dacron" fabric, made in a tubular form, held by multifilament threads of the same material that in its manufacture forms a kind of nucleus of said ring c and which fasten same to support a.

A fundamental part in the obtainment of said prosthesis and its characteristics is due to its manufacturing process, particularly that regarding support a and the textile ring c that holds the unit in position by means of sutures.

Said support a is manufactured in one single piece without casting or welding and without any deformation of the alloy used due to forging, temperature or any other system that can negatively modify the properties of the metal.

For this purpose the alloy bar is machined and cast by means of the Laser ray so as to shape within the internal portion of the annular body 1 thus obtained, on one side the middle arm 3 and on the other the arms 5, said casting process being effected on both sides to obtain this result. Subsequently, the piece is shaped by means of electroerosion, thus obtaining the final shape of said arms, the piece is finished with a final polish and manual adjustment.

It is hereby pointed out that the portion affected by temperature dissipation is superficial and that same is eliminated by means of the finishing process, without affecting the alloy of the piece obtained.

The occluding disc b is basically formed by a carbon substrate, graphite, known as isotropic pyrolitic carbon, that has a coating that consists of a special bath of thermal deposit carbon which offers the best characteristics for this type of valve due to its biocompatibility with blood, particularly its thromboresistance togetehr with high resistance to degradation due to use and fatigue in the biologic means.

Said disc b is assembled on support a in a floating adaptation between support arms 3 and 5, with freedom to rotate, that allows to alternate the contact surfaces of same with terminals 4 and 6 of said arms, which avoids the possibility of wear and/or fatigue in fixed points of said disc b.

The suture textile ring c is made by cutting an adequate measure of a "Dacron" fabric tube, obtained in said tubular form in adequate circular machines, placed on the external portion of support a as a cover, over the perimetral groove 2 of same.

Subsequently, five turns with a multifilament thread, also of "Dacron", are given on the fabric around said groove 2 conveniently knotting same at intervals of each turn. In this way the textile material, by means of the thread and the knots, is held to support a. Then the perimetral edges of the tubular fabric a_re joined together by means of a longitudinal seam, that is to say, perimetral to the support, the safety thread remaining in this way inside the ring thus formed and covered by same.

The fabric is then dampened and withdrawn from support a, turning the ring inside out so that the above mentioned longitudinal seam is turned to the inside. Said ring that is kept damp is again placed over groove 2 of support a by means of a dilator and finally, the unit is placed in an oven at an adequate temperature in order to obtain the memory of the fabric in the compression or shrinking process of same. Thus, on producing the physical phenomenon of shrinkage when it is dried, the textile ring c is fixed on support a     with the assisting retention supplied by the perimetral edges that delimit groove 2 of the housing of same.

It is pointed out that the assembly of the occluding disc b and that of the suture textile ring c on support a may be effected indistinctly first one and then the other, same not varying the final finishing result of the valve.

In said assembly, support a with the occluding disc b in position are rotable with respect to the textile ring c, that is to say, there is a possibility that said textile ring c may slip, that allows the surgeon to place said valve in the desired position once the textile ring c is sutured in its place.

It must be pointed out that once the valve has been implanted, the subsequent celular coverage that penetrates the fabric completes the desired biologic effect for fixing.

It is doubtless that in putting the  present invention into practice some modifications may be introduced as regards certain details, without this involving a departure from the basic principles which are clearly specified in the following
claims.

## C L A I M S

Having especially described and determined the nature of the present invention and the manner in which same shall be put into practice, I hereby claim as my exclusive right and property:

1. A cardiovascular valvular prosthesis, of the type that comprises a suture textile ring arranged surrounding the perimetral groove of a support on which an occluding disc is assembled, characterized by the fact that said support is constituted by an integral piece, of annular shape, on one of the edges of which is projected a radially directed support arm that ends in a terminal directed toward the internal portion of same, said arm is complemented with a pair of projections symmetrically arranged with respect to same, that face the opposite edge of said annular piece according to a virtual cord of same determined between the diameter of the piece and the beginning of the above mentioned radially directed arm; between the terminals of said arms and of said projections the occluding disc is adapted, free to rotate, that on the surface facing the above mentioned arm has a circular depression the edge of which forms a support step of the above mentioned terminal of said arm; the textile ring adapted in the outside portion of said perimetral groove of the support is constituted by a length of a tubular piece of fabric that has its perimetral edges joined by means of longitudinal seam disposed against said groove.

2. A cardiovascular valvular prosthesis, as claimed in claim 1, characterized by the fact that the support of said occluding disc is constituted by an integral annular piece, machined on a cobalt based alloy, the internal edges of which have a support arm radially directed that being shorter than the internal radius of said annular piece, has a terminal directed toward the interior of same; from the opposite edge jut out a pair of projections that are directed according to a virtual cord, normal to said support arm, determined between the beginning of same and the diameter of the support; the terminals of said arm and of said projections define three single tangential contact points between said occluding disc and its pivot support between which said disc is floatingly adapted free to rotate; said disc has one of its faces substantially convex and its opposite surface, that faces the terminal of the support arm, has a circular recess the edge of which defines a support step of said terminal, said recess is framed in a portion that converges with the substantially convex opposite face, in a perimetral edge of a curvilinear transversal section; on the external perimetral groove shaped on the annular support is adapted, with possibility of slipping on same, a suture textile ring being constituted by a length of tubular fabric, with the edges joined by a longitudinal seam adapted against said groove, the internal portion of which has a safety thread winding that helps to hold said textile ring.

3. A cardiovascular valvular prosthesis, as claimed in any of claims 1 and 2, characterized by the fact that said occluding disc is made based on isotropic pyrolitic carbon that has a certain tungsten percentage of its radiopaque, said disc has a coating that consists of a carbon cover thermically deposited on same; said disc being of a slightly lower diameter than that of its perimetral support in the annular support in the closed position, defines a slight peripheric deficiency that determines a washing action of said support and at the same time capable of avoiding a static accumulation of blood in said closed position of the valve.

4. A process to manufacture the cardiovascular valvular prosthesis, as claimed in claims 1 to 3, characterized by the fact that an alloy bar based on cobalt is machined to the adequate dimension; the machined piece is cast by means of a Laser ray thus forming, on the one hand a support arm radially oriented and on the other a pair of projections according to a virtual cord between the diameter and the beginning of the above mentioned arm; model said piece to obtain the final shape of said arm and said projections and finish giving the unit a final adjustment; separately form an occluding disc, the dimensions of which are adequate to the internal diameter of the above mentioned machined piece, with a substantially convex face the opposite face having a circular central recess, floatingly adaptable between the terminals of the above mentioned support arm and the projections symmetrically arranged on both sides of same; cut an adequate length of tubular fabric, of a diameter corresponding to the external diameter of the machined support piece of the occluding disc, cover the external groove formed by machining said piece, wind a thread on same adequately knotting it at intervals of each turn, take the perimetral edges of said length of tubular fabric and join by means of a longitudinal seam, then dampen the sewn fabric withdrawing same from the support, turn the textile ring thus formed inside out in order to leave the above mentioned longitudinal seam toward its inner portion, place said damp ring again in position on the above mentioned support groove and, finally, dry the unit to obtain the recuperation by shrinkage of the fabric of the ring with its internal wound thread.

5. A process to manufacture a cardiovascular valvular prosthesis as claimed in claim 4, characterized by the fact that the occluding disc, being formed on isotropic pyrolitic carbon, is coated with a thermically deposited bath of carbon on same.

6. A cardiovascular valvular prosthesis and manufacturing process of same, in accordance with the above mentioned claims and the attached specifications and drawings.

0050763

Fig.1

Fig.2

0050763

**Fig.3**

**Fig.4**

**Fig.5**

**Fig.6**